**Europäisches Patentamt**

**European Patent Office**

**Office européen des brevets**

(11) Publication number: **0 171 771**
**B1**

(12) **EUROPEAN PATENT SPECIFICATION**

(45) Date of publication of patent specification: **28.02.90**

(21) Application number: **85110047.9**

(22) Date of filing: **09.08.85**

(51) Int. Cl.⁵: **C 12 N 7/02** // A61K39/205

(54) **A method for purification of rabies virus.**

(30) Priority: **10.08.84 JP 168226/84**

(43) Date of publication of application:
**19.02.86 Bulletin 86/08**

(45) Publication of the grant of the patent:
**28.02.90 Bulletin 90/09**

(84) Designated Contracting States:
**AT BE CH DE FR GB IT LI NL SE**

(56) References cited:
**DE-A-2 159 863**
**FR-A-2 344 629**
**FR-A-2 499 412**
**NL-A- 131 092**
**US-A-3 105 012**

**VIROLOGY, vol. 11, no. 3, July 1960, pages
554,560,562,568,569; PHILIPSON et al.: "The
purification and concentration of viruses by
aqueous polymer phase systems"**

(73) Proprietor: **Juridical Foundation The Chemo-
Sero-Therapeutic Research Institute
668, Okubo Shimizu-machi
Kumamoto-shi Kumamoto-ken (JP)**

(72) Inventor: **Sakamoto, Kuniaki
160-5, Miyukifueta-machi
Kumamoto-shi Kumamoto-ken (JP)**
Inventor: **Ohkuma, Kunio
313-20, Okubo Shimizu-machi
Kumamoto-shi Kumamoto-ken (JP)**
Inventor: **Kawahara, Tetsuo
402, Hokamaki Ohzu-machi
Kikuchi-gun Kumamoto-ken (JP)**
Inventor: **Sakoh, Mitsuo
119-5, Kaminogou-machi
Kumamoto-shi Kumamoto-ken (JP)**

(74) Representative: **Vossius & Partner
Siebertstrasse 4 P.O. Box 86 07 67
D-8000 München 86 (DE)**

Courier Press, Leamington Spa, England.

**Description**

The present invention relates to a method for the purification of rabies virus, and particularly to such method for obtaining effective vaccines against rabies.

Rabies is a disease occuring almost all over the world, particularly in Asian and African countries. This desease is caused by rabies virus, which is known to be a bullet-shaped RNA virus belong to *Rhabdoviridae* measuring about 180 nm in length and about 80 nm in diameter. The virus is infective to mammalian animals. Thus, rabies is mediated even through wild animals such as bats, foxes, weasels etc. as well as dogs. Affliction with rabies occurs upon the invasion of rabies virus into the central nervous system through peripheral nerves and is transmitted from an animal infected with the virus by biting or licking. The mortality rate in human patients approaches almost 100 per cent.

The only possible way for preventing and curing such horrible disease is a vaccination, in which a highly purified vaccine is desired to be used.

A typical conventional method for the production of a rabies vaccine, particularly for animals, includes the step of preparing a rabies virus-containing material by propagating rabies virus infected into the brain of mice or any other appropriate animals and harvesting the propagated virus from the brain and the step of refining or purifying the harvested material by means of centrifugation and/or chemical treatments with such agent as sodium carboxymethyl cellulose, followed by an inactivation treatment. More recently, particularly in the case of production of rabies vaccine for human beings, tissue-culture types of rabies vaccines have been developed in which there are used such cells as cultured chick embryo cells. This type of vaccine is more effective and safe because there are contained less contaminants as would originate from the brain substances. A typical dried inactivated tissue culture rabies vaccine of such type is prepared as follows:

```
                 Chick embryo cell
                        |
       Inoculation of rabies virus into the cell
                        |
       Cultivation of the virus (35°C, 5 - 7 days)
                        |
        Harvest of the virus (membrane filtration)
                        |
             Inactivation of the virus
                        |
  (β-propiolactone 0.04% by volume,heating at 37°C
                        |
      for 60 minutes, repeated two times)
                        |
           Concentrating by ultrafiltration
                        |
           Ultra-high-speed centrifugation
                        |
            ┌───────────┴───────────────┐
        Precipitate                  Supernatant
            |
   Treatment with sodium chloride-containing
            |
      buffer solution (M/100, pH 7.1)
            |
             Conditioning
            |
             Final bulk
            |
            Freeze drying
```

However, the conventional methods for preparing rabies vaccine, including the one as illustrated by the above flow diagram, require sophisticated and costly techniques, such as ultrafiltration and ultrahigh-speed centrifugation, to purify the rabies virus and only produce vaccines of low purities.

It is therefore the object of the present invention to provide a method by which rabies virus can be purified in a simple and unexpensive manner to produce a rabies vaccine of high purity.

The present invention is based on the discovery that a sulfuric acid ester of cellulose or a crosslinked polysaccharide has a specific affinity with rabies virus, and is effective for isolation and purification of the virus from a material containing the same. Thus, according to the present invention, there is provided a method for the purification of rabies virus which comprises subjecting a solution containing the rabies virus to column chromatography using, as a gel for chromatography, a sulfuric acid ester of a crosslinked polysaccharide or cellulose.

The sulfuric acid ester of cellulose to be used in the present invention includes a sulfuric acid ester of crystalline cellulose or cellulose having crystalline area and non-crystalline area. These starting celluloses are commercially available, for example, as Abicel (manufactured by Asahi Kasei in Japan), Cellulofine GC—15, GH—25, GC—100, or GC—200 (manufactured by Chisso Corp. in Japan).

The sulfuric acid ester of a crosslinked polysaccharide to be used in the present invention includes a sulfuric acid ester of polysaccharides, such as dextran, cellulose, agarose, which is crosslinked with a crosslinked agent, such as epichlorohydrin, dichlorohydrin dibromohydrin, ethylene glycol bisepoxypropyl ether. The crosslinked polysaccharides are commercially available, for example, as crosslinked dextran such as Sephadex G—10, G—25, G—50, and G—100 (manufactured by Pharmacia in Sweden), crosslinked agaroses such as Sepharose C1—2B, C1—4B, and C1—6B (manufacutred by Pharmacia in Sweden), and crosslinked celluloses such as Cellulofine GCL—25, GCL—90 (manufactured by Chisso Corp. in Japan).

The sulfation of such crosslinked polysaccharide or cellulose can be carried out by a conventional method. However, the gel for chromatography to be used in the present invention is characterized in that it is prepared by directly sulfating cellulose or a crosslinked polysaccharide, which are water-insoluble, with a sulfating agent such as chlorosulfonic acid or anhydrous sulfuric acid in an organic solvent (e.g. pyridine). Thus, the resultant gel is water-insoluble and highly stable. Further, such gel of the sulfuric acid ester of cellulose or a crosslinked polysaccharide exhibits an extremely high adsorbing activity since it is fully sulfated, even at the inner regions thereof. The use of the gel is also advantageous from an economical standpoint, because it can be easily prepared at a low cost. The degree of sulfation (content of the sulfonyl group) of crosslinked polysaccharide is usually in the range of 0.1 to 40%, preferably 10 to 40%, based on the weight of the crosslinked polysaccharide, and the degree of sulfation of cellulose is usually in the range of 0.1 to 5.0%, based on the cellulose.

The procedure of purification of rabies virus by column chromatography using the sulfuric acid ester of a crosslinked polysaccharide or cellulose is carried out in a similar manner to that in conventional column chromatography. For instance, the method is carried out in the following manner: Firstly, a sulfuric ester of a crosslinked polysaccharide or cellulose (preferably, in the form of spherical particles) is packed within a column, which is equilibrated with a suitable buffer solution, preferably having an ionic strength of about 0.001 to 2.0, for example, 0.01 M phosphate buffered saline solution containing 0.14 M NaCl (pH 7.0—8.0). After the equilibration, a rabies virus-containing solution to be treated is passed through the column in order to adsorb such virus onto the gel, followed by washing with the same buffer solution as used for the above equilibration. Thereafter, the adsorbed rabies virus is eluted from the column by passing through the column a suitable buffer solution having an ionic strength larger than that of the buffer solution used for the equilibration or the washing, for example, 1.0 M or 1.5 M sodium chloride-containing phosphate buffer solution (pH 6—9) to give desired highly purified rabies virus.

The method of the present invention can be applied to any solution containing rabies virus and can be conducted at any stage in the purification of rabies virus. Thus, the method can be applied to a solution containing rabies virus before being subjected to an inactivation treatment as well as an inactivated rabies virus-containing solution. For example, a solution just harvested from the cultured chick embryo cell as mentioned above can undergo the method of the present invention in order to purify or isolate the rabies virus contained therein. An inactivated rabies virus-containing solution, such as one obtained by the inactivation with β-propiolactone as mentioned or with ultraviolet rays, can also be subjected to the method of the invention for purifying the virus. The present invention can be of course applied to a rabies virus-containing solution produced by any other process, for example, those obtained by propagating rabies virus infected into the brain of mice or any other animals. The method of the present invention may also be applied to a solution containing rabies virus proteins which would be expressed by means of genetic engineering.

According to the purification method of the present invention rabies virus can be purified to a high degree, with the least contamination with proteins, lipids and other substances resulting from the cell used and/or the culturing medium. This is probably due to the fact that the sulfonyl group bonds directly to the crosslinked polysaccharide or cellulose in the sulfuric acid ester of a crosslinked polysaccharide or cellulose and hence it has a high content of sulfonyl group and shows excellent specific adsorbability to rabies virus. The purification method of the present invention can be easily done with simple operation without need for expensive equipment give the desired purified rabies virus on an industrial scale with lower cost. If desired, the method of the present invention can be combined with conventional separation techniques (e.g. ultra-centrifugation) so as to obtain rabies virus as highly as possible.

The present invention will now be illustrated by the following Preparations (preparations of gels for chromatography) and Examples.

EP 0 171 771 B1

### Preparation 1

To pyridine (600 ml) is added dropwise chlorosulfonic acid (117 g) at below 0°C. After the addition, the mixture is heated to 65—70°C. To the mixture is added crystalline cellulose gel (Cellulofine GC—15, manufactured by Chisso Corp.) (80 g), and the mixture is stirred at 65—70°C for 3 hours. After the completion of the reaction, the mixture is cooled and neutralized with 10% aqueous sodium hydroxide. The gel thus obtained is separated by filtration and washed well with 0.01 M phosphate buffer-aqueous sodium chloride mixture to give a cellulose sulfate gel.

### Preparation 2

To pyridine (600 ml) is added dropwise chlorosulfonic acid (117 g) at below 0°C. After the addition, the mixture is heated to 65—70°C. To the mixture is added crystalline cellulose (Abicel for chromatography, manufactured by Asahi Kasei) (80 g), and the mixture is stirred at 65—70°C for 4 hours. After the completion of the reaction, the reaction mixture is cooled and then neutralized with 10% aqueous sodium hydroxide. The gel thus obtained is separated by filtration and washed well with 0.01 M phosphate buffer-aqueous sodium chloride mixture to give a cellulose sulfate gel.

### Preparation 3

To pyridine (200 ml) is added dropwise chlorosulfonic acid (11 ml) at below 0°C. After the addition, the mixture is heated to 65—70°C. To the mixture is added epichlorohydrin-crosslinked dextran (Sephadex G—50, manufactured by Pharmacia) (7.5 g), and the mixture is stirred at 65—70°C for 4 hours. After the reaction, the reaction mixture is cooled and then neutralized with aqueous sodium hydroxide. The gel thus obtained is separated by filtration and washed well with 0.01 M phosphate-buffered saline solution to give a crosslinked dextran sulfate.

### Example 1

The cellulose sulfate gel obtained in the manner as described in Preparation 1 is packed within a column (25 mmφ × 400 mm), followed by the passage of 1,400 ml of distilled water through the column. The packed column is equilibrated with 0.01 M phosphate-buffered saline solution containing 0.14 M sodium chloride. Then, through the column is passed, at a rate of 500 ml/minute, 2,900 ml of an inactivated rabies virus-containing solution obtained by using chick embryo cells followed by an inactivation treatment, as described in the above. After the passage of the virus containing solution, the column is fully washed with 0.01 M phosphate buffer solution containing 0.14 M sodium chloride. Then, the adsorbed material is eluted with 500 ml of 0.01 M phosphate buffer solution containing 1.0 M sodium chloride (specific conductivity 87.6 mS/cm, pH 7.3) at a rate of 1 ml/minute. The starting solution and the eluate are determined with respect to the respective virus contents in terms of HA (Hemagglutinin) titer. Determination is also made with respect to the amounts of the contaminant proteins resulted from the cell used, in comparison with the conventional method in which the inactivated virus-containing solution is purified by the ultrafiltration and the ultrahigh-speed centrifugation. The results are shown in Table 1, which demonstrates that the virus contained in the starting solution is sufficiently recovered by the method of the invention, and the degree of purification by the method of the present invention, in terms of the amount of the contaminant proteins, is approximately two times as high as that by the conventional method.

TABLE 1

| Virus Content (HA titer) | | Contaminant Proteins | |
|---|---|---|---|
| Starting Solution | Eluate | Conventional Method | Present Invention |
| 185,600 | 335,360 | 0.027 mg/ml | 0.014 mg/ml |

### Example 2

Using the cellulose gel obtained in Preparation 2, a procedure is conducted similar to Example 1 except that 4,200 ml of the inactivated rabies virus-containing solution is passed through the column and the adsorbed material is eluted with 0.01 M phosphate buffer solution containing 1.5 M NaCl (specific conductivity 120 mS/cm, pH 7.2). The results are summarized in Table 2.

4

TABLE 2

| Virus Content (HA titer) | | Contaminant Proteins | |
|---|---|---|---|
| Starting Solution | Eluate | Conventional Method | Present Invention |
| 134,000 | 204,800 | 0.020 mg/ml | 0.014 mg/ml |

## Example 3

Using the crosslinked dextran sulfate gel, a purification procedure is conducted in a similar manner to that in Example 1, except that a solution just harvested from the cultured chick embryo cells (a solution prior to the inactivation) is used in an amount of 3,000 ml. The virus content and the amounts of the contaminant proteins are determined in the same manners as in Example 1, with the results shown in Table 3.

TABLE 3

| Virus Content (HA titer) | | Contaminant Proteins | |
|---|---|---|---|
| Starting Solution | Eluate | Conventional Method | Present Invention |
| 105,600 | 176,500 | 0.028 mg/ml | 0.015 mg/ml |

## Claims

1. A method for the purification of rabies virus, which comprises subjecting a solution containing the rabies virus to a column chromatography using, as a gel for chromatography, a sulfuric acid ester of cellulose or a cross-linked polysaccharide, said sulfuric acid ester being prepared by sulfating cellulose or a cross-linked polysaccharide with a sulfating agent in an organic solvent.

2. The method according to claim 1, wherein the rabies virus-containing solution is one obtained by propagating rabies virus using a culture of chick embryo cells followed by an inactivation of the virus.

3. The method according to claim 1, wherein the rabies virus-containing solution is one just harvested from the cultured medium of a culture of chick embryo cells.

4. The method according to claim 1, wherein the rabies virus-containing solution is one obtained by propagating rabies virus infected into the brain of mice or any other appropriate animals.

5. The method according to any one of claims 1 to 4, wherein the sulfuric acid ester of a cross-linked polysaccharide is a cross-linked cellulose sulfate, a cross-linked agarose sulfate or a cross-linked dextran sulfate.

6. The method according to any one of claims 1 to 4, wherein the sulfuric acid ester of cellulose is a sulfuric acid ester of crystalline cellulose or of a cellulose having crystalline and non-crystalline areas.

## Patentansprüche

1. Verfahren zur Reinigung von Tollwutvirus, bei dem man eine Tollwutvirus-enthaltende Lösung einer Säulenchromatographie unter Verwendung eines Schwefelsäureesters von Cellulose oder eines quervernetzten Polysaccharids als Chromatographiegel unterzieht, wobei der Schwefelsäureester durch Sulfonierung von Cellulose oder eines quervernetzten Polysaccharids mit einem Sulfonierungsmittel in einem organischen Lösungsmittel hergestellt wird.

2. Verfahren nach Anspruch 1, wobei die Tollwutvirus-enthaltende Lösung durch Vermehrung von Tollwutvirus unter Verwendung einer Hühnerembryonen-Zellkultur und nachfolgende Inaktivierung des Virus erhalten wird.

3. Verfahren nach Anspruch 1, wobei die Tollwutvirus-enthaltende Lösung frisch aus dem Kulturmedium einer Hühnerembryonen-Zellkultur geerntet wird.

4. Verfahren nach Anspruch 1, wobei die Tollwutvirus-enthaltende Lösung durch Vermehrung von in das Gehirn von Mäusen oder anderen geeigneten Tieren infiziertem Tollwutvirus erhalten wird.

5. Verfahren nach einem der Ansprüche 1 bis 4, wobei der Schwefelsäureester eines quervernetzten Polysaccharids quervernetztes Cellulosesulfat, quervernetztes Agarosesulfat oder quervernetztes Dextransulfat ist.

6. Verfahren nach einem der Ansprüche 1 bis 4, wobei der Schwefelsäureester von Cellulose ein Schwefelsäureester von kristalliner Cellulose oder von Cellulose mit kristallinen und nicht-kristallinen Bereichen ist.

**Revendications**

1. Procédé de purification du virus de la rage, caractérisé en ce que l'on soumet une solution contenant le virus de la rage à une chromatographie sur colonne en utilisant, à titre de gel pour la chromatographie, un ester de l'acide sulfurique de la cellulose, ou un polysaccharide réticulé, ledit ester de l'acide sulfurique étant préparé en sulfatant la cellulose ou un polysaccharide réticulé avec un agent sulfatant dans un solvant organique.

2. Procédé suivant la revendication 1, caractérisé en ce que la solution contenant le virus de la rage en est une que l'on obtient par la propagation du virus de la rage en se servant d'une culture de cellules d'embryons de poussins, suivie de l'inactivation du virus.

3. Procédé suivant la revendication 1, caractérisé en ce que la solution contenant le virus de la rage en est une que l'on vient juste de récolter du milieu de culture provenant d'une culture de cellules d'embryons de poussins.

4. Procédé suivant la revendication 1, caractérisé en ce que la solution contenant le virus de la rage en est une obtenue par la propagation du virus de la rage dont on a infecté le cerveau de souris, ou de n'importe quels autres animaux appropriés.

5. Procédé suivant l'une quelconque des revendications 1 à 4, caractérisé en ce que l'ester de l'acide sulfurique d'un polysaccharide réticulé est un sulfate de cellulose réticulé, un sulfate d'agarose réticulé, ou un sulfate de dextrane réticulé.

6. Procédé suivant l'une quelconque des revendications 1 à 4, caractérisé en ce que l'ester de l'acide sulfurique de la cellulose est un ester de l'acide sulfurique de cellulose cristalline, ou d'une cellulose possédant des zones cristallines et non cristallines.